# DEMANDE DE BREVET EUROPEEN

(11) **EP 4 112 122 A1**
(43) Date de publication de la demande: **04.01.2023**
(21) Numéro de dépôt: 21020344.4
(22) Date de dépôt: 02.07.2021
(51) Int. Cl.: A61N 5/10

(54) **OUTIL DE RÉGLAGE POUR RÉTICULE D'ACCÉLÉRATEUR LINÉAIRE DE RADIOTHÉRAPIE**

(71) Demandeur: Dux, Martin, 1728 Rossens FR (CH)
(72) Inventeur: Dux, Martin, 1728 Rossens FR (CH)

(57) **Abrégé**

Outil de réglage pour réticule d'accélérateur linéaire de radiothérapie.

L'outil de réglage pour réticule d'accélérateur linéaire de radiothérapie comporte un cadre 1, quatre trou 3, une poignée 2, six socles 6 équipés chacun d'une glissière cylindrique 4 et d'une tige filetée 5. Une fois le cadre inséré dans le porte-accessoire de l'accélérateur de radiothérapie, les poussoirs 8 et 9 sont insérés dans les glissières cylindriques. Les poussoirs 8 et 9 sont équipés chacun d'un roulement linéaire 10 ainsi que d'une plaque 11. Six écrous à ailettes 14 sont vissés sur la tige filetée de chaque socle après l'installation des poussoirs 8 et 9. Le positionnement du réticule est réalisé avec les écrous à ailettes. Lorsque le positionnement est jugé parfait, les quatre trous 3 permettent de serrer les vis du réticule.

## Description

### Objet et/ou domaine technique

La présente invention concerne un outil de réglage pour réticule d'accélérateur linéaire Varian^{®} de radiothérapie ("crosshair adjustment tool" en anglais et "Fadenkreuz Einstellung werkzeug" en allemand)

### Description de l'invention

### État de la technique

L'ajustement du réticule d'un accélérateur linéaire Varian^{®} est particulièrement délicat. Le réglage est réalisé en poussant les bords du réticule avec un tournevis. Ainsi même si le réticule est bien positionné lors du réglage, il est fréquent qu'au resserrage des vis le réticule se déplace et ne soit plus parfaitement aligné avec les mâchoires, ou ne soit plus à l'isocentre.

### Inconvénients

Le réglage du réticule prend beaucoup de temps si l'on désire qu'il soit parfaitement à l'isocentre et simultanément parallèle aux mâchoires. De plus, le résultat est rarement parfait.

### Problème technique

Le but de la présente invention est de permettre un réglage du réticule parfaitement à l'isocentre et parfaitement aligné avec les mâchoires de l'accélérateur Varian^{®}

### Solution

L'outil inventé se glisse dans le porte-accessoire présent sur chaque accélérateur linéaire Varian^{®}.

Les caractéristiques décrites dans la revendication 2 permettent de résoudre le problème technique

### Avantages

L'invention permet un réglage du réticule parfaitement à l'isocentre et parfaitement aligné avec les mâchoires de l'accélérateur Varian^{®}.

### Énumération des figures/dessins

L'invention va être exposée de manière plus détaillée à l'aide d'un exemple de réalisation représenté dans les dessins suivants:
Fig. 1: Outil de réglage sans les poussoirs
Fig. 2: Outil de réglage avec les poussoirs
Fig. 3 : Détail de la fig. 2

### Réalisation de l'invention

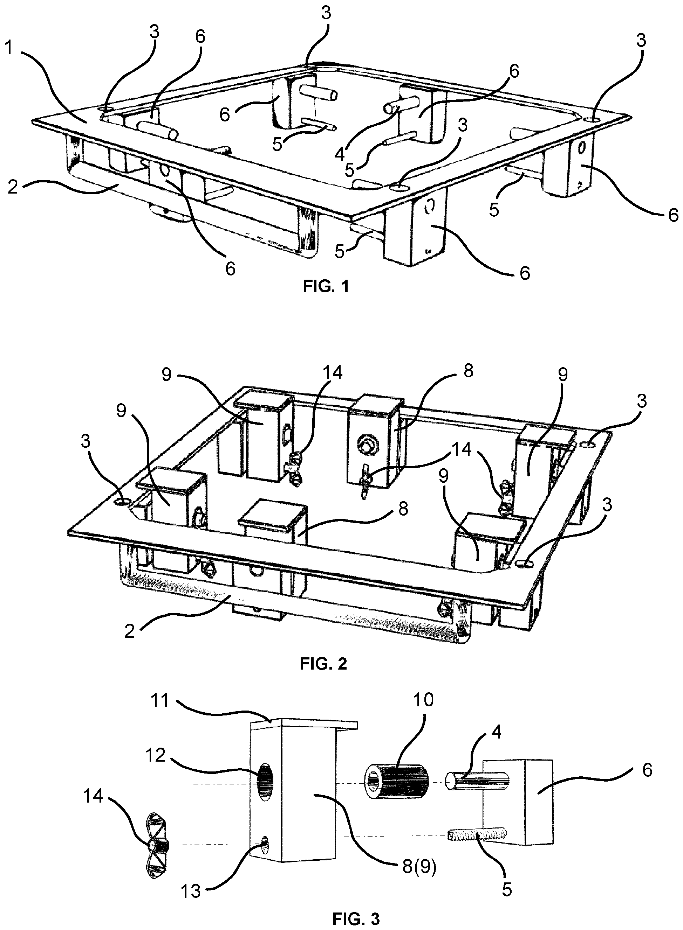

Fig.1, Six socles 6 sont fixés sur un cadre 1 de même dimension que les accessoires Varian^{®}.Chaque socle 6 est équipé d'une tige filetée 5 et d'une glissière cylindrique 4. Le cadre 1 comporte également une poignée 2. Dans le cadre 1, quatre trous 3 permettent de dévisser le réticule de l'accélérateur et de le revisser une fois le réglage effectué.
Fig. 2 Une fois que le cadre 1 est inséré dans le porte-accessoire Varian^{®}, les quatre poussoirs 9 et les deux poussoirs 8 sont introduits dans les six socles 6. L'écrou à ailettes 14 est vissé sur la tige filetée 5 des six socles 6. Ces écrous à ailettes 14 permettent de pousser les poussoirs 8 et 9 pour un positionnement précis du réticule.
Fig. 3 Une barre cylindrique 4 et une tige filetée 5 sont fixées sur le socle 6. Un roulement linéaire 10 est inséré dans le trou 12 sur le poussoir 8 ou 9. Le trou 13 permet à la tige filetée 5 de traverser le bloc poussoir. La plaque 11 est fixée sur le poussoir 8 ou 9. L'écrou à ailettes 14 est vissé une fois le poussoir 8 ou 9 installé sur le socle 6.

## Revendications

1. Utilisation du porte-accessoire de l'accélérateur pour insérer l'outil de réglage du réticule.

2. La figure 2 représente l'outil lorsqu'il est inséré dans le porte accessoire de l'accélérateur Varian^{®}.L'outil est composé d'un cadre 1, d'une poignée 2, de six socles 6 et de six poussoirs 8 et 9 permettant de positionner le réticule. Quatre poussoirs 9 sont utilisés pour permettre l'alignement avec les mâchoires «X» de l'accélérateur et centrer le réticule sur l'axe «X» de l'accélérateur et deux poussoirs 8 pour centrer le réticule sur l'axe «Y» de l'accélérateur .

3. La figure 1 représente l'outil de réglage sans les poussoirs qui sont retirés afin de glisser l'outil dans le porte-accessoire de l'accélérateur.

4. Le cadre 1 comporte également quatre trous 3 permettant de serrer les vis du réticule de l'accélérateur lorsque le réglage est effectué.

5. Six socles 6, pourvus chacun d'une glissière cylindrique 4 et d'une tige filetée 5, sont fixés sur le cadre 1.

6. La tige filetée 5 montée sur chaque socles est équipé d'un écrou à ailettes 14 permettant d'appuyer sur les poussoirs 8 et 9 afin de positionner le réticule de l'accélérateur.

7. Les poussoirs 8 et 9 sont équipés chacun d'un roulement linéaire 10 permettant leur déplacement sans friction.
